# FASCICULE DE BREVET EUROPEEN

(11) **EP 3 204 763 B1**
(45) Date de publication et mention de la délivrance du brevet: **12.12.2018**
(21) Numéro de dépôt: 15791689.1
(22) Date de dépôt: 06.10.2015
(51) Int. Cl.: G01N 29/032, B06B 3/00, G01N 29/22, C22B 9/02, C22B 21/06, C22C 21/04, C22C 21/08, G01N 29/02, G01N 29/34, G01N 33/20

(54) **PROCEDE DE MOUILLAGE D'UNE SONOTRODE ET SONOTRODE**
VERFAHREN ZUR BEFEUCHTUNG EINER SONOTRODE UND SONOTRODE
METHOD FOR WETTING A SONOTRODE AND SONOTRODE

(30) Priorité: 07.10.2014 FR 1402256
(43) Date de publication de la demande: 16.08.2017
(73) Titulaire: Constellium Issoire, 63500 Issoire (FR)
(72) Inventeur: ACHARD, Jean-Louis, F-38220 Vizille (FR); JARRY, Philippe, F-38500 Grenoble (FR)
(74) Mandataire: Constellium - Propriété Industrielle
(86) Numéro de dépôt international: PCT/FR2015/052679
(87) Numéro de publication internationale: WO 2016/055728

(56) Documents cités:
- GB-A- 1 487 203
- JP-A- 2007 239 102
- US-A1- 2012 042 751

## Description

La présente invention concerne un procédé d'utilisation d'une sonotrode, notamment une sonotrode en céramique, dans de l'aluminium et/ou du magnésium liquide. Selon un deuxième aspect, la présente invention concerne un dispositif d'insonification comprenant ladite sonotrode.

Dans le domaine de la coulée des métaux et plus particulièrement de l'aluminium liquide, il est de la plus haute importance de contrôler la qualité du métal liquide, par exemple par la détermination de la propreté inclusionnaire. D'elle dépend en effet la qualité et le taux de mise au rebus des tôles minces préparées à partir de l'aluminium liquide pour la fabrication d'articles, en particulier de récipients fermés du type boite de boisson ou aérosol. Le cas échéant, il est nécessaire d'appliquer des traitements adéquats du type dégazage, filtration en vue de la réduction du nombre d'inclusions, etc..., pour améliorer la qualité du métal avant d'effectuer la coulée.

Des méthodes ultrasonores pour la mesure et le traitement des métaux liquides à bas point de fusion (Ga, Sn, Pb, Zn...) ont été développées depuis les années 1960. Ces méthodes utilisent des guides d'onde métalliques couplés avec les métaux liquides pour l'émission et la réception d'ondes, grâce au mouillage des guides d'onde obtenu respectivement par les métaux à analyser.

Le contrôle non destructif en ligne dans l'aluminium liquide et son traitement se heurte au caractère très réactif de ce dernier. Le mouillage par l'aluminium liquide de métaux utilisés comme guides d'ondes ultrasonores (aciers, titane, etc.) conduit en effet à la dissolution de ces métaux, de sorte que les mesures effectuées dans l'aluminium liquide ne sont pas fiables dans la durée.

Qui plus est, le mouillage, même dans ce cas, n'est pas parfait, et des méthodes ont été développées pour l'améliorer. En témoigne notamment le brevet EP0035545B1, sous priorité de 1979, de « Reynolds Metal Company » revendiquant le dépôt d'un film d'aluminium en phase vapeur sur une sonotrode en titane. Mais en fait, même dans une telle configuration, la qualité du mouillage évolue en cours d'utilisation du fait de la réaction de la matière du guide d'ondes avec l'aluminium liquide.

Par ailleurs, l'utilisation de guides d'ondes en céramique réfractaire n'est pas optimale du fait de l'absence de mouillage des céramiques par l'aluminium empêchant un bon couplage entre la sonotrode et le métal liquide. Une interface mouillante entre la céramique et l'aluminium pourrait être créée par dépôt préalable chimique d'un métal sur le guide d'onde mais cette méthode de dépôt est couteuse et le dépôt en question fugace.

Un des buts de l'invention est de développer une interface stable entre le guide d'onde et l'aluminium liquide, évitant toute dissolution du matériau constituant le guide d'onde et ne nécessitant pas la réalisation d'un dépôt d'interface. A cet effet, la présente invention propose un procédé d'utilisation d'une sonotrode comprenant les étapes suivantes :
a) Fournir un premier bain d'un métal liquide comprenant de l'aluminium avec une teneur X et du magnésium avec une teneur Y, la teneur Y en magnésium étant différente de zéro,
b) Plonger au moins en partie une sonotrode formée en un matériau inerte à l'aluminium liquide, dans le premier bain de métal liquide, et
c) Appliquer des ultrasons de puissance à la sonotrode de sorte à exciter le métal liquide jusqu'à l'obtention d'un mouillage de la sonotrode par le métal liquide.
d) Refroidir le premier métal du premier bain jusqu'à l'obtention de la solidification du premier métal liquide autour de la sonotrode, générant une liaison intime entre la sonotrode et le premier métal liquide solidifié présentant une force de liaison sensiblement égale à celle d'un brasage entre deux métaux.
e) Usiner le premier métal solidifié sous la forme d'une bride configurée pour la fixation d'un amplificateur mécanique et /ou d'un transducteur.

Ainsi, grâce à ce procédé, la sonotrode, une fois émergée du premier bain de métal liquide, a montré la présence d'une couche stable en aluminium et/ou de magnésium, c'est-à-dire qui ne peut pas être pelée comme c'est ordinairement le cas pour une céramique simplement plongée dans l'aluminium liquide, démontrant l'existence d'un mouillage de la sonotrode.

De plus, la sonotrode ayant subi ce traitement et utilisée ensuite dans de l'aluminium liquide a montré sa capacité à transmettre et réceptionner des ondes ultrasonores de faible puissance dans le domaine du MHz, communément appelées ultrasons de mesure, prouvant également l'obtention d'un mouillage.

De préférence, l'étape a) consiste à fournir un premier bain de métal liquide comprenant du magnésium avec une teneur Y supérieure ou égale à 0,05%, de préférence une teneur Y supérieure à 0,5%, et mieux encore une teneur Y supérieure ou égale à 0,7% en poids.

L'association d'une petite quantité de magnésium à l'aluminium liquide permet en effet d'obtenir un bon mouillage de la sonotrode par l'aluminium. Le mouillage est d'autant plus rapidement obtenu que la teneur en magnésium du métal liquide est importante. La durée d'excitation est notamment maintenue pendant plusieurs minutes pour des teneurs en magnésium inférieures à 1% et moins d'une minute pour une présence de Mg d'environ 5%.

Selon une possibilité, l'étape a) consiste à fournir un premier bain de métal liquide dans lequel la teneur X en aluminium liquide est nulle. Dans cette variante, la sonotrode est alors mouillée par du magnésium. Le procédé offre ainsi une alternative au procédé de mouillage d'une sonotrode par le magnésium, qui est généralement obtenu à partir d'une sonotrode en un matériau en titane ou en acier. Ceci élargit les matériaux envisageables de sonotrode pour un mouillage avec du magnésium et les champs d'application.

Avantageusement, la sonotrode plongée à l'étape b) est formée en une céramique de nitrure de silicium ou d'oxynitrure de silicium, notamment un SiAlON. Il est entendu dans le présent document que le terme SiAlON provient de l'acronyme anglosaxon de « Silicon-Aluminum-Oxygen-Nitride », définissant une famille de céramiques réfractaires à base de silicium, d'aluminium, d'azote et d'oxygène, également définie comme des oxynitrures de silicium et d'aluminium. L'obtention d'un mouillage avec le SiAlON, matériau connu pour être inerte vis-à-vis de l'aluminium liquide et largement utilisé dans l'industrie de l'aluminium, est d'autant plus surprenante que même après des durées de mises en contact prolongées, l'aluminium n'adhère pas aux tubes de SiAlON utilisés comme gaines de thermoplongeurs. La preuve en est que la couche d'aluminium qui reste après émersion des gaines de SiAlON est facilement pelée.

Selon une disposition, l'étape c) d'application d'ultrasons de puissance consiste à appliquer des ultrasons de basse fréquence. De préférence, la fréquence utilisée est comprise entre 10 et 40kHz. La puissance des ultrasons appliquée est par exemple comprise entre 50 et 150W. Ces fréquences sont typiquement adaptées pour exciter la sonotrode et l'aluminium liquide. D'autres puissances sont bien entendues utilisables pour peu qu'elles permettent la génération d'une cavitation dans le métal liquide en un temps compatible avec les contraintes industrielles, typiquement de quelques minutes.

Par exemple, pour une teneur Y en magnésium de 2,5% en poids, avec des ultrasons d'une fréquence d'environ 20KHz et une puissance de 150W, la durée d'excitation nécessaire est inférieure à 10 minutes pour obtenir le mouillage de la sonotrode.

Selon une possibilité, l'étape a) comprend la fourniture d'un premier bain de métal liquide comprenant de l'aluminium avec une teneur X différente de zéro de sorte que le premier bain comprend un premier alliage d'aluminium liquide.

Le procédé comprend, après l'étape c), une étape d) de refroidissement du premier alliage d'aluminium liquide du premier bain jusqu'à l'obtention de la solidification du premier alliage d'aluminium autour de la sonotrode, générant une liaison intime entre la sonotrode et le premier alliage d'aluminium solidifié.

Grace à ce procédé, la sonotrode est liée intimement à un alliage d'aluminium solidifié, avec des propriétés similaires à celles obtenues lors d'un brasage entre deux métaux. Une section polie de l'interface obtenue par ce procédé entre la sonotrode liée à l'aluminium, observée par microscope électronique à balayage (MEB) montre en effet un scellement avec une liaison parfaite, sans aucune décohésion et une continuité métallurgique entre les deux matériaux du type à permettre un couplage mécanique optimal entre l'aluminium et la sonotrode.

Ainsi, la liaison intime entre la sonotrode et le premier alliage d'aluminium solidifié présente une force de liaison au moins sensiblement égale à celle d'un brasage entre deux métaux.

Toujours selon cette possibilité, le procédé comprend une étape e), réalisée après l'étape d), d'usinage du premier alliage d'aluminium solidifié sous la forme d'une bride configurée pour la fixation d'un amplificateur mécanique et /ou d'un transducteur. Bien entendu cette étape e) d'usinage de l'aluminium solidifé est réalisée après l'avoir dégagé du creuset. Cette configuration permet d'obtenir un couplage mécanique sans décohésion malgré la transmission d'ultrasons de puissance pendant plusieurs heures en continu. Aucun signe de découplage n'apparait entre le transducteur vissé à la bride et la sonotrode. Cette configuration permet de grandement améliorer la qualité de l'assemblage formé classiquement entre la sonotrode et le transducteur à l'aide d'une bride métallique simplement serrée sur la sonotrode. Cet assemblage de l'art antérieur est en effet inapte à transmettre des puissances élevées pendant des durées supérieures à quelques minutes.

Selon une variante, le procédé comprend après l'étape e), les étapes de
f) Immersion de l'autre extrémité de la sonotrode (3) et mouillage dans le premier bain de métal liquide (1) de ladite sonotrode (3) comme à l' étape c),
g) Fourniture d'un second bain d'un second alliage d'aluminium liquide,
h) Immersion au moins en partie de la sonotrode dans le second bain du second alliage d'aluminium liquide, et
i) Application d'ultrasons de puissance à la sonotrode pour régénérer le mouillage
j) Application d'ultrasons de puissance ou de mesure à la sonotrode

La sonotrode intialement mouillée dans le premier alliage d'alumimium est en effet réutilisable dans un autre bain d'aluminium distinct du premier bain. Dans ce cas, il est préférable d'appliquer à nouveau des ultrasons de puissance à la sonotrode pour reactiver le mouillage. En effet, le fait d'émerger la sonotrode mouillée du premier bain dans une atmosphère non anhydre génèrerait la formation d'un oxyde en surface, gênant la transmission des ultrasons. L'application d'ultrasons de puissance dans le second bain permet alors la régénération du mouillage, même en l'absence de magnésium dans ce dernier. Dans cette variante, la bride permettant la fixation du transducteur à la sonotrode peut être formée selon les étapes d) et e) du procédé précédemment décrites.

Dans ce cas, le second alliage d'aluminium liquide fourni à l'étape g) comprend du magnésium avec une teneur Y' comprise entre 0 et 0,7% en poids.

Selon une possibilité le second alliage d'aluminium liquide du second bain fourni à l'étape g) est formé d'un alliage de AISiMg liquide, l'alliage d'AISiMg liquide comprenant du Si avec une teneur de 0,5 à 7% en poids et du Mg avec une teneur Y' de 0 à 0,7% en poids de Mg. Il est ainsi possible d'utiliser une sonotrode mouillée grâce à l'invention pour transmettre de façon durable et efficace des ultrasons de puissance pour le traitement de tout type d'alliage d'aluminium liquide ou des ultrasons de mesure pour le contrôle non destructif de l'alliage.

Selon une autre variante, le second bain comprend tout type d'alliage d'aluminium liquide, comme par exemple du type AlCuMg.

Lorsque la teneur X en aluminium liquide du premier bain de métal liquide est nulle, le mouillage de la sonotrode est alors réalisé par le magnésium ce qui permet toutefois une utilisation ultérieure pour véhiculer des ultrasons dans un second bain d'alliage d'aluminium.

Les ultrasons appliqués à l'étape i) du procédé, lorsque le second bain d'alliage d'aluminium diffère du premier bain de métal liquide, présentent notamment une fréquence de vibration comprise entre 10 et 40kHz, de préférence environ 20 kHz, et par exemple avec une puissance comprise entre 50 et 150W, de préférence environ 150W, pendant une durée de quelques minutes, et de préférence environ 10 minutes.

Selon un second aspect, l'invention concerne un dispositif d'insonification comprenant au moins une sonotrode en céramique, formée en nitrure de silicium ou en oxynitrure de silicium, tel qu'un SiAlON, et une bride en alliage d'aluminium fixée par une liaison intime à la sonotrode. Ce dispositif assure un couplage mécanique durable entre un transducteur et la sonotrode et rend ensisageables des mesures de qualité de l'aluminium liquide. Ce dispositif peut par ailleurs être utilisé en combinaison avec le procédé de mouillage de la sonotrode en SiAlON par l'aluminium liquide de sorte à améliorer la fiabilité des mesures en contrôle non destructif de l'aluminium liquide, telles que la détection d'inclusions, la velocimétrie Doppler aux ultrasons, l'hydrophonie dans l'aluminium liquide.

D'autres aspects, buts et avantages de la présente invention apparaîtront mieux à la lecture de la description suivante d'un mode de réalisation de celle-ci, donnée à titre d'exemple non limitatif et faite en référence aux dessins annexés. Les figures ne respectent pas nécessairement l'échelle de tous les éléments représentés de sorte à améliorer leur lisibilité. Dans la suite de la description, par souci de simplification, des éléments identiques, similaires ou équivalents des différentes formes de réalisation portent les mêmes références numériques.
- Les figures 1 à 3 sont une illustration du schéma de principe du procédé d'utilisation d'une sonotrode selon un mode de réalisation de l'invention.
- Les figures 4 à 6 sont une illustration du schéma de principe de la formation d'une liaison intime selon un mode de réalisation de l'invention.
- Les figures 7 à 8 sont une illustration du schéma de principe d'une autre utilisation de la sonotrode mouillée selon un mode de réalisation de l'invention.

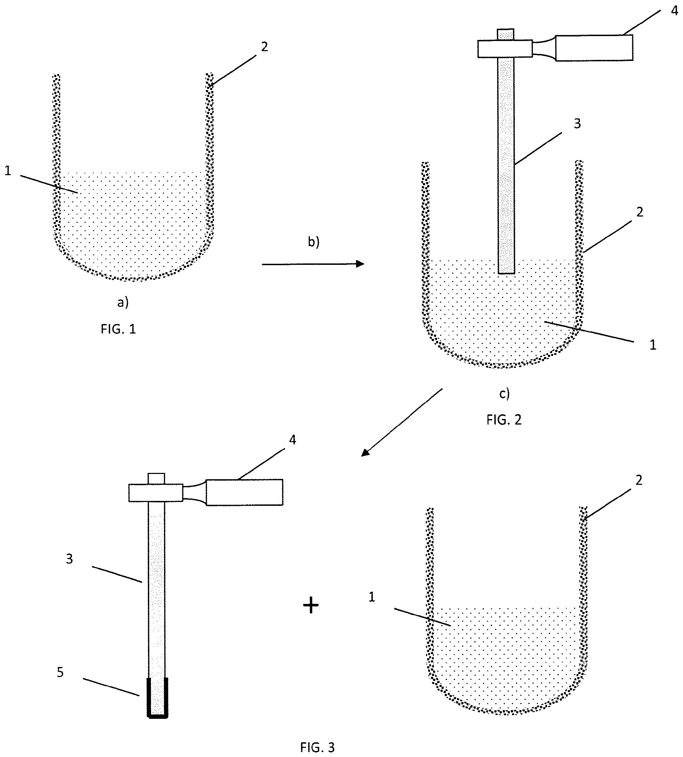

Comme illustré à la figure 1, un premier bain d'un métal liquide 1 est préparé dans un récipient 2 tel qu'un creuset. Le métal liquide 1 comprend notamment une teneur X non nulle en aluminium liquide (composant majoritaire) et une teneur Y en magnésium d'environ 0.7% en poids selon l'étape a) du procédé. Une sonotrode 3 en céramique de SiAlON, réfractaire et inerte à l'aluminium liquide, est ensuite plongée en partie dans le premier bain de métal liquide 1 (étape b) figure 2). Des ultrasons de puissance sont appliquées par l'intermédiaire d'un transducteur 4 ou un ensemble transducteur-amplificateur à la sonotrode 3 de sorte à l'exciter (étape c). Les ultrasons de puissance appliqués sont des ultrasons de basse fréquence, de l'ordre de 20 kHz avec une puissance de 150W. Après quelques minutes de ce traitement un mouillage 5 de la sonotrode 3 se forme, une couche ou pellicule d'aluminium non facilement pelable est en effet retrouvée en surface de la sonotrode 3 (figure 3). D'autres fréquences de vibration dite « de basse fréquence » notamment comprises entre 10 et 40kHz peuvent être utilisées pour exciter le métal liquide 1 via la sonotrode 3. De même, d'autres valeurs de puissance sont envisageables dans la mesure où elles sont suffisantes pour générer le phénomène de cavitation dans le métal liquide en un temps compatible avec les procédés industriels de sorte à obtenir un mouillage rapidement.

Selon une variante de réalisation non illustrée, la teneur Y en magnésium dans le métal liquide 1 est de 0.05, ou de 0.5 % en poids. La durée du traitement par ultrasons pour l'obtention du mouillage est alors prolongée par comparaison avec celle obtenue pour une teneur Y en magnésium de 0.7% en poids.

Selon une alternative, la même durée de traitement pour l'obtention du mouillage que celle obtenue avec une teneur Y en magnésium de 0.7% en poids est réalisée grâce à l'augmentation de la puissance des ultrasons.

Par ailleurs, selon une autre variante, toute sonotrode 3 constituée d'un matériau inerte à l'aluminium liquide tel qu'une céramique réfractaire de la famille de nitrure de silicium ou d'oxynitrure de silicium est mouillée par l'aluminium liquide grâce à ce procédé.

Selon encore une autre variante non illustrée, la teneur X en aluminium du premier bain 1 est nulle. Dans ce cas, le mouillage de la sonotrode 3 est obtenu par le magnésium dont la teneur Y avoisine 100% en poids. Cette sonotrode 3 ainsi mouillée est alors plongée dans un second bain d'un second alliage d'aluminium liquide pour être ensuite apte à véhiculer des ultrasons sans perte de puissance sur une longue période.

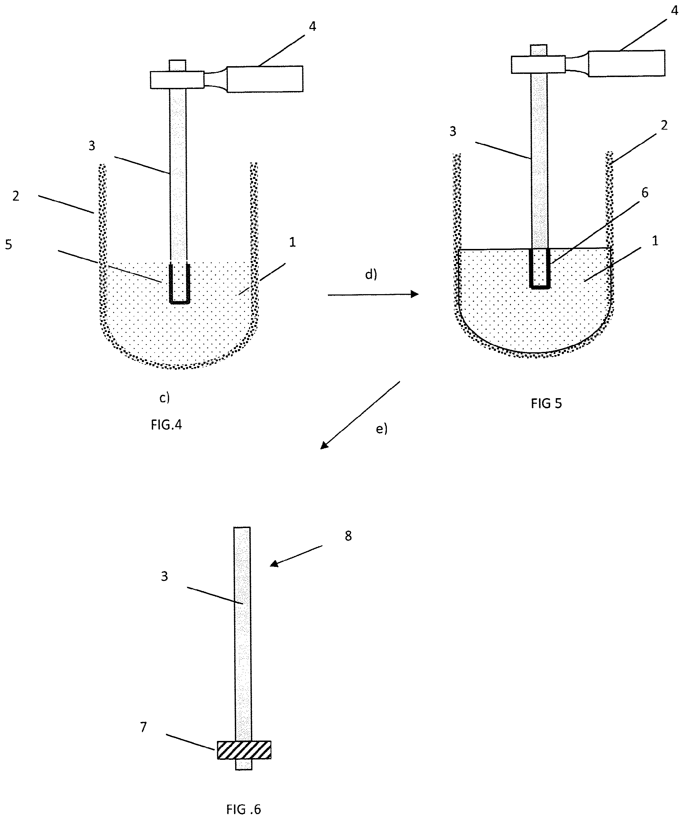

Selon une possibilité illustrée aux figures 4 à 6, le premier bain comprenant le premier alliage d'aluminium liquide 1, dans lequel la sonotrode 3 est mouillée par application des ultrasons de puissance (figure 4 - étape c), est laissé revenir à température ambiante (figure 5 - étape d). Une fois refroidi, le premier alliage d'aluminium 1 est solidifié autour de la sonotrode 3, générant une liaison intime 6 entre la sonotrode 3 et l'alliage solidifié 1 (visible à la figure 5). La liaison intime 6 correspond à un scellement avec une liaison parfaite, sans aucune décohésion et une continuité métallurgique entre la céramique et le premier alliage d'aluminium. Puis, la sonotrode 3 intimement liée au premier alliage d'aluminium 1 est dégagée du creuset 2 et l'alliage 1 solidifié est usiné par tournage et perçage de sorte à former une bride 7 cylindrique liée intimement avec la céramique (figure 6, étape e). La liaison intime 6 entre l'aluminium et le SiAlON est formée avec des propriétés similaires à celles obtenues lors d'un brasage entre deux métaux. Un dispositif d'insonification 8 est ainsi obtenu, il permet un couplage mécanique optimal entre l'aluminium et la sonotrode 3.

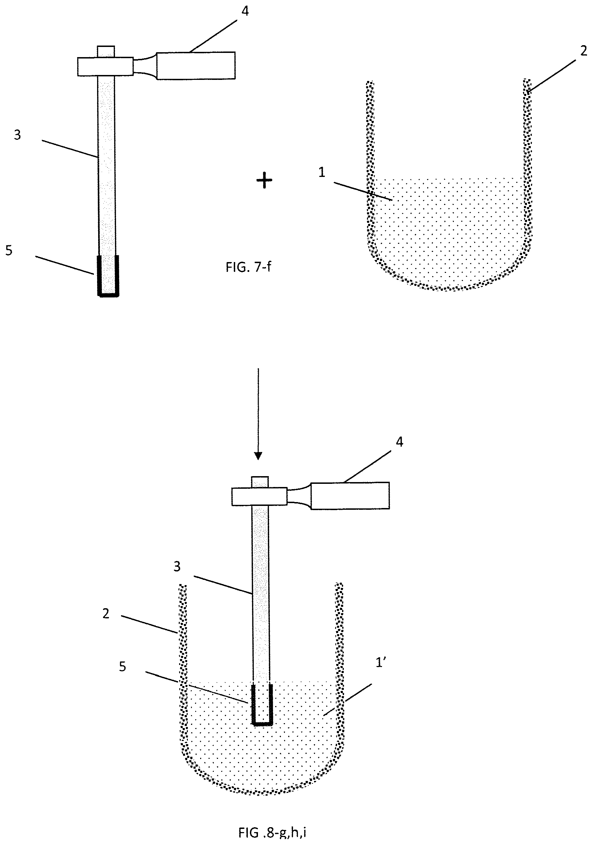

Bien entendu, ce procédé peut être mis en oeuvre à partir d'alliages d'aluminium et de magnésium de compositions différentes, avec ou sans silicium et notamment à partir d'un alliage comportant du cuivre. Selon une possibilité illustrée sur les figures 7 et 8, la sonotrode 3 mouillée par de l'aluminium liquide, est retirée du premier bain 1 (étape f, figure 7) pour être en partie immergée dans un second bain d'un second alliage d'aluminium liquide 1' (étape g, h, figure 8). Des ultrasons sont appliqués à la sonotrode 3 avec une fréquence de vibration de 20 kHz et une puissance d'environ 150W de sorte à régénérer le mouillage 5 (étape i) même en absence de magnésium dans le second alliage d'aluminium liquide 1'. Le second alliage d'aluminium liquide 1' est en effet formé d'AISiMg avec une teneur comprise entre 0,5 et 7% en poids de Si et une teneur Y de 0% à 0,7% en poids de Mg. La sonotrode 3 ainsi obtenue peut alors être à nouveau utilisée pour transmettre efficacement les ultrasons de puissance, ou de mesure (avec fréquence de 100 kHz par exemple) dans le second alliage d'aluminium liquide 1'.

Selon une possibilité non illustrée, la bride 7 en liaison intime 6 avec la sonotrode 3 à l'issue de l'étape f), figure 6, du procédé est ensuite utilisée pour la fixation d'un transducteur 4. La sonotrode 3 ainsi rattachée au transducteur 4 est alors mouillée dans un bain d'un alliage d'aluminium tel précédemment décrit. Les ultrasons émis par le transducteur 4 sont alors transmis par l'intermédiaire de la bride 7 à la sonotrode 3 mouillée qui à son tour transmet les ultrasons au bain de l'alliage d'aluminium en vue d'effectuer des contrôles ou des traitements de l'aluminium liquide pour une coulée de qualité.

Ainsi, la présente invention propose un procédé d'utilisation d'une sonotrode 3 par un mouillage obtenu à partir de l'aluminium ou du magnésium, qui est peu coûteux et simple à réaliser. L'invention propose également la formation d'une liaison intime 6 entre le matériau de la sonotrode et un alliage d'aluminium 1' solidifié très simple à mettre en oeuvre et permettant de fabriquer une bride 7 parfaitement scellée à la sonotrode 3, et utilisable sur la durée notamment pour transmettre des ultrasons de mesure ou de puissance.

Il va de soi que l'invention n'est pas limitée au mode de réalisation décrit ci-dessus à titre d'exemple mais qu'elle comprend tous les équivalents techniques et les variantes des moyens décrits ainsi que leurs combinaisons.

## Revendications

1. Procédé d'utilisation d'une sonotrode (3) comprenant les étapes suivantes :
a) Fournir un premier bain d'un métal liquide (1) comprenant de l'aluminium avec une teneur X et du magnésium avec une teneur Y, la teneur Y en magnésium étant différente de zéro,
b) Plonger au moins en partie une sonotrode (3) formée en un matériau inerte à l'aluminium liquide, dans le premier bain de métal liquide (1),
c) Appliquer des ultrasons de puissance à la sonotrode (3) de sorte à exciter le métal liquide (1) jusqu'à l'obtention d'un mouillage (5) de la sonotrode (3) par le métal liquide (1),
d) Refroidir le premier métal liquide (1) du premier bain jusqu'à l'obtention de la solidification du premier métal liquide (1) autour de la sonotrode (3), générant une liaison intime (6) entre la sonotrode (3) et le premier métal liquide (1) solidifié présentant une force de liaison sensiblement égale à celle d'un brasage entre deux métaux.
e) Usiner le premier métal (1) solidifié sous la forme d'une bride (7) configurée pour la fixation d'un amplificateur mécanique et /ou d'un transducteur (4) .

2. Procédé selon la revendication 1, dans lequel l'étape a) consiste à fournir un premier bain de métal liquide (1) comprenant du magnésium avec une teneur Y supérieure ou égale à 0,05%, de préférence une teneur Y supérieure à 0,5%, et mieux encore une teneur Y supérieure ou égale à 0,7% en poids.

3. Procédé selon l'une des revendications 1 à 2, dans lequel la sonotrode (3) plongée à l'étape b) est formée en une céramique de nitrure de silicium ou d'oxynitrure de silicium, notamment un SiAlON.

4. Procédé selon l'une des revendications 1 à 3, dans lequel l'étape c) d'application d'ultrasons de puissance consiste à appliquer des ultrasons de basse fréquence, de préférence d'une fréquence comprise entre 10 et 40 kHz.

5. Procédé selon l'une des revendications 1 à 4, dans lequel l'étape a) consiste à fournir un premier bain de métal liquide (1) dans lequel la teneur X en aluminium liquide est nulle.

6. Procédé selon l'une des revendications 1 à 4, dans lequel l'étape a) comprend la fourniture d'un premier bain de métal liquide (1) comprenant de l'aluminium avec une teneur X différente de zéro de sorte que le premier bain comprend un premier alliage d'aluminium (1) liquide.

7. Procédé selon l'une des revendications 1 à 6, comprenant, après l'étape e), les étapes de
f) Immersion de l'autre extrémité de la sonotrode (3) et mouillage dans le premier bain de métal liquide (1) de ladite sonotrode (3) comme à l'étape c)
g) Fourniture d'un second bain d'un second alliage d'aluminium (1') liquide,
h) Immersion au moins en partie de la sonotrode (3) dans le second bain du second alliage d'aluminium (1') liquide,
i) Application d'ultrasons de puissance à la sonotrode (3) pour régénérer le mouillage,
j) Application d'ultrasons de puissance ou de mesure à la sonotrode (3).

8. Procédé selon la revendication 7, dans lequel le second alliage d'aluminium (1') liquide fourni à l'étape g) comprend du magnésium avec une teneur Y' comprise entre 0 et 0,7% en poids.

9. Procédé selon la revendication 7 ou 8, dans lequel le second alliage d'aluminium (1') liquide du second bain fourni à l'étape g) est formé d'un alliage de AlSiMg (1') liquide, l'alliage d'AlSiMg (1') liquide comprenant du Si avec une teneur de 0,5 à 7% en poids et du Mg avec une teneur Y' de 0 à 0,7% en poids de Mg.

10. Dispositif d'insonification (8) comprenant au moins une sonotrode (3) en céramique, formée en nitrure de silicium ou en oxynitrure de silicium, tel qu'un SiAlON, et une bride (7) en un premier alliage d'aluminium (1), fixée par une liaison intime (6) à la sonotrode (3) obtenue par le procédé selon la revendication 6.

## Patentansprüche

1. Verfahren zur Verwendung einer Sonotrode (3), das folgende Schritte aufweist:
a) Bereitstellen eines ersten Bades aus einem Flüssigmetall (1), das Aluminium mit einem Gehalt X und Magnesium mit einem Gehalt Y aufweist, wobei der Gehalt Y an Magnesium von null verschieden ist,
b) Zumindest teilweises Eintauchen einer Sonotrode (3) aus einem gegenüber flüssigem Aluminium inerten Material in das erste Flüssigmetallbad (1),
c) Anwenden von Leistungsultraschall auf die Sonotrode (3) zur Erregung des Flüssigmetalls (1), bis eine Benetzung (5) der Sonotrode (3) mit dem Flüssigmetall (1) erhalten wird,
d) Abkühlen des ersten Flüssigmetalls (1) des ersten Bades bis zur Erstarrung des ersten Flüssigmetalls (1) um die Sonotrode (3) herum, wodurch zwischen Sonotrode (3) und erstarrtem ersten Flüssigmetall (1) eine enge Verbindung (6) mit einer Verbindungskraft entsteht, die im Wesentlichen gleich der Verbindungskraft beim Löten zweier Metalle ist,
e) Bearbeiten des erstarrten ersten Metalls (1) in der Form eines Flansches (7), der zur Befestigung eines mechanischen Verstärkers und/oder eines Wandlers ausgestaltet ist.

2. Verfahren nach Anspruch 1, bei dem in Schritt a) ein erstes Flüssigmetallbad (1) bereitgestellt wird, aufweisend Magnesium mit einem Gehalt Y größer oder gleich 0,05 Gew.-%, bevorzugt mit einem Gehalt Y größer 0,5 Gew.-%, und besonders bevorzugt mit einem Gehalt Y größer oder gleich 0,7 Gew.-%.

3. Verfahren nach einem der Ansprüche 1 bis 2, bei dem die in Schritt b) eingetauchte Sonotrode (3) aus einer Keramik aus Siliziumnitrid oder Siliziumoxynitrid, insbesondere SiAlON, gebildet ist.

4. Verfahren nach einem der Ansprüche 1 bis 3, bei dem im Schritt c) der Anwendung von Leistungsultraschall niederfrequenter Ultraschall angewendet wird, vorzugsweise mit einer Frequenz im Bereich zwischen 10 und 40 kHz.

5. Verfahren nach einem der Ansprüche 1 bis 4, bei dem in Schritt a) ein erstes Flüssigmetallbad (1) bereitgestellt wird, in dem der Gehalt X an flüssigem Aluminium null ist.

6. Verfahren nach einem der Ansprüche 1 bis 4, bei dem der Schritt a) das Bereitstellen eines ersten Flüssigmetallbades (1) umfasst, das Aluminium mit einem Gehalt X verschieden von null aufweist, so dass das erste Bad eine erste flüssige Aluminiumlegierung (1) aufweist.

7. Verfahren nach einem der Ansprüche 1 bis 6, das nach dem Schritt e) folgende Schritte aufweist:
f) Eintauchen des anderen Endes der Sonotrode (3) und Benetzen der Sonotrode (3) im ersten Flüssigmetallbad (1) wie in Schritt c),
g) Bereitstellen eines zweiten Bades aus einer zweiten flüssigen Aluminiumlegierung (1'),
h) Zumindest teilweises Eintauchen der Sonotrode (3) in das zweite Bad aus der zweiten flüssigen Aluminiumlegierung (1'),
i) Anwenden von Leistungsultraschall auf die Sonotrode (3) zur erneuten Benetzung,
j) Anwenden von Leistungs- oder Messultraschall auf die Sonotrode (3).

8. Verfahren nach Anspruch 7, bei dem die in Schritt g) bereitgestellte zweite flüssige Aluminiumlegierung (1') Magnesium mit einem Gehalt Y' im Bereich zwischen 0 und 0,7 Gew.-% aufweist.

9. Verfahren nach Anspruch 7 oder 8, bei dem die zweite flüssige Aluminiumlegierung (1') des in Schritt g) bereitgestellten zweiten Bades von einer flüssigen AlSiMg-Legierung (1') gebildet ist, wobei die flüssige AlSiMg-Legierung (1') Si mit einem Gehalt von 0,5 bis 7 Gew.-% und Mg mit einem Gehalt Y' von 0 bis 0,7 Gew.-% aufweist.

10. Beschallungsvorrichtung (8) umfassend mindestens eine Sonotrode (3) aus Keramik, gebildet aus Siliziumnitrid oder Siliziumoxynitrid wie SiAlON, und einen durch eine enge Verbindung (6) an der Sonotrode (3) befestigten Flansch (7) aus einer ersten Aluminiumlegierung (1), der mit dem Verfahren nach Anspruch 6 hergestellt wird.

## Claims

1. Method for using a sonotrode (3) comprising the following steps:
a) Providing a first bath of a liquid metal (1) comprising aluminium with a content X and magnesium with a content Y, the magnesium content Y being different to zero,
b) Immersing at least partially a sonotrode (3) formed from a material inert to liquid aluminium, in the first bath of liquid metal (1),
c) Applying power ultrasounds to the sonotrode (3) so as to excite the liquid metal (1) until wetting (5) of the sonotrode (3) by the liquid metal (1) is obtained,
d) Cooling the first liquid metal (1) of the first bath until solidification of the first liquid metal (1) around the sonotrode (3) is obtained, generating an intimate bond (6) between the sonotrode (3) and the solidified first liquid metal (1) having a bonding strength substantially equal to that of brazing between two metals.
e) Machining the solidified first metal (1) in the form of a flange (7) configured for the attachment of a mechanical amplifier and/or of a transducer (4).

2. Method according to claim 1, wherein step a) consists of providing a first bath of liquid metal (1) comprising magnesium with a content Y greater than or equal to 0.05%, preferably a content Y greater than 0.5%, and more preferably a content Y greater than or equal to 0.7% by weight.

3. Method according to one of claims 1 to 2, wherein the sonotrode (3) immersed in step b) is formed from a silicon nitride or silicon oxynitride ceramic, particularly a SiAlON.

4. Method according to one of claims 1 to 3, wherein step c) for applying power ultrasounds consists of applying low-frequency ultrasounds, preferably of a frequency between 10 and 40 kHz.

5. Method according to one of claims 1 to 4, wherein step a) consists of providing a first bath of liquid metal (1) wherein the liquid aluminium content X is zero.

6. Method according to one of claims 1 to 4, wherein step a) comprises the provision of a first bath of liquid metal (1) comprising aluminium with a content X different to zero such that the first bath comprises a first liquid aluminium alloy (1).

7. Method according to one of claims 1 to 6, comprising, after step e), steps for
f) Immersing the other end of the sonotrode (3) and wetting in the first bath of liquid metal (1) said sonotrode (3) as in step c)
g) Providing a second bath of a second liquid aluminium alloy (1'),
h) Immersing at least partially the sonotrode (3) in the second bath of the second liquid aluminium alloy (1'),
i) Applying power ultrasounds to the sonotrode (3) to regenerate the wetting,
j) Applying power or measurement ultrasounds to the sonotrode (3).

8. Method according to claim 7, wherein the second liquid aluminium alloy (1') provided in step g) comprises magnesium with a content Y' between 0 and 0.7% by weight.

9. Method according to claim 7 or 8, wherein the second liquid aluminium alloy (1') of the second bath provided in step g) is formed from a liquid AlSiMg alloy (1'), the liquid AlSiMg alloy (1') comprising Si with a content of 0.5 to 7% by weight and Mg with a content Y' of 0 to 0.7% by weight of Mg.

10. Insonification device (8) comprising at least a sonotrode (3) made of ceramic, formed from silicon nitride or silicon oxynitride, such as a SiAlON, and a flange (7) made of a first aluminium alloy (1), attached by an intimate bond (6) to the sonotrode (3) obtained using the method according to claim 6.
